# EUROPEAN PATENT APPLICATION

(11) **EP 3 982 699 A1**
(43) Date of publication of application: **13.04.2022**
(21) Application number: 20819236.9
(22) Date of filing: 01.06.2020
(51) Int. Cl.: H05H 1/26, A61N 1/44, A61B 18/00

(54) **PLASMA IRRADIATION APPARATUS AND PLASMA IRRADIATION METHOD**

(30) Priority: 04.06.2019 JP 2019104559
(71) Applicant: NGK SPARK PLUG CO., LTD., Mizuho-ku Nagoya-shi, Aichi 467-8525 (JP)
(72) Inventor: ITOH Shinsuke, Nagoya-shi, Aichi 467-8525 (JP)
(74) Representative: Zimmermann & Partner Patentanwälte mbB
(86) International application number: PCT/JP2020/021655
(87) International publication number: WO 2020/246436

(57) **Abstract**

A plasma irradiation apparatus (20) includes: a gas guide channel (30) defining therein a flow path (36) for flow of a discharge gas, with an outlet port (34) formed at an end of the flow path (36); and a discharge section that generates plasma discharge in the gas guide channel (30). The plasma irradiation apparatus is mounted to a distal device (3, 203, 303) which is movable relative to a target substance. The plasma irradiation apparatus (20) further includes a valve (72) arranged in the gas guide channel (30) or a gas supply channel and configured as a valve capable of changing a flow rate of the discharge gas according to an opening degree of the valve or as a check valve.

## Description

### Technical Field

The present invention relates to a plasma irradiation apparatus and a plasma irradiation method.

### Background Art

Patent Documents 1 and 2 each disclose an apparatus for performing plasma discharge. The apparatuses disclosed in each of Patent Documents 1 and 2 is configured to supply a discharge gas through a gas guide channel and generate a plasma by changing a voltage between a plurality of electrodes arranged in proximity to the gas guide channel.

### Prior Art Documents

### Patent Document

Patent Document 1: Japanese Translation of PCT International Application Publication No. 2013-544122
Patent Document 2: Japanese Translation of PCT International Application Publication No. 2014-519875

### Summary of the Invention

### Problems to be Solved by the Invention

In conventional plasma irradiation apparatuses including those disclosed in Patent Documents 1 and 2, however, there are cases where plasma discharge is performed in a state that a large amount of impurity gas (such as air) different from the discharge gas exists in the gas guide channel. These conventional plasma irradiation apparatuses face the possibility that, when plasma discharge is performed in a state that a large amount of impurity gas exists in the gas guide channel, the plasma discharge may not take place stably or a plasma different in properties from the intended plasma may be generated.

The present invention has been accomplished to solve at least part of the above-mentioned problems. It is an object of the present invention to provide a technique for preventing plasma discharge from being performed in a state that a large amount of impurity gas exists in a gas guide channel.

### Means for Solving the Problems

According to one aspect of the present invention, there is provided a plasma irradiation apparatus comprising:
a gas guide channel defining therein a flow path for flow of a discharge gas, with an outlet port formed at an end of the flow path;
a discharge section having a first electrode, a second electrode and a dielectric layer interposed between the first electrode and the second electrode and being operable to generate plasma discharge in the gas guide channel; and
a gas supply channel defining therein a path for supply of the discharge gas to the gas guide channel, the gas supply channel being disposed at a location closer to the gas guide channel than a gas control section which executes flow rate control of the discharge gas,
the plasma irradiation apparatus being adapted for mounting on a distal device which is movable relative to a target substance,
wherein the plasma irradiation apparatus further comprises a check valve arranged in the gas guide channel or the gas supply channel to allow the discharge gas to flow in a first direction toward the outlet port and prevent the discharge gas from flowing in a second direction reverse to the first direction.

In this plasma irradiation apparatus, the check valve is arranged in the gas guide channel or the gas supply channel so as to allow the discharge gas to flow in a first direction toward the outlet port and prevent the discharge gas from flowing in a second direction reverse to the first direction. By the arrangement of such a check valve, a large amount of impurity gas (such as air) different from the discharge gas is prevented from flowing into piping upstream of the check valve (i.e. piping closer to the gas control section than the check valve). As a result, the occurrence of unstable discharge due to the entry of a large amount of impurity gas is suppressed.

According to another aspect of the present invention, there is provided a plasma irradiation apparatus comprising:
a gas guide channel defining therein a flow path for flow of a discharge gas, with an outlet port formed at an end of the flow path;
a discharge section having a first electrode, a second electrode and a dielectric layer interposed between the first electrode and the second electrode and being operable to generate plasma discharge in the gas guide channel; and
a gas supply channel defining therein a path for supply of the discharge gas to the gas guide channel,
the plasma irradiation apparatus being adapted for mounting on a distal device which is movable relative to a target substance,
wherein the plasma irradiation apparatus further comprises:
   a valve arranged in the gas guide channel or the gas supply channel to control a flow rate of the discharge gas according to an opening degree of the valve;
   a discharge control section that controls the discharge section to perform plasma discharge; and
   a valve control section that controls opening and closing of the valve, and
   wherein the valve control section is configured to:
      keep the valve opened during at least a discharge period in which the discharge control section allows the discharge section to perform the plasma discharge; and
      open the valve during a predetermined preparation period, in which the discharge control section prohibits the discharge section from performing the plasma discharge, before the discharge period.

In this plasma irradiation apparatus, the valve is kept opened during at least the "discharge period" in which the discharge control section allows the discharge section to perform the plasma discharge; and the valve is also opened during the "predetermined preparation method before the discharge period" in which the discharge control section prohibits the discharge section from performing the plasma discharge. In other words, the discharge gas is kept flowing in the gas guide channel even during the "predetermined preparation period before the discharge period". By this control, any impurity gas flowing back from the outside into the gas guide channel is discharged out during the preparation period. It is thus unlikely that a large amount of impurity gas will exist in the gas guide channel at the time of start of the discharge period subsequent to the preparation period. As a consequence, the plasma discharge is prevented from being performed in a state that a large amount of impurity gas exists in the gas guide channel.

In the above-mentioned plasma irradiation apparatus, the valve control section may be configured to open the valve at a larger opening degree during at least a partial period in the preparation period than that during the discharge period.

By this valve control, the discharge gas flows more vigorously into the gas guide channel during at least the partial period in the preparation period. Thus, the impurity gas in the gas guide channel is discharged out more effectively during the preparation period.

In the above-mentioned plasma irradiation apparatus, the valve control section may be configured to continuously or intermittently open the valve at a smaller opening degree during a rest period from an end of the discharge period to a start of the next preparation period than that during the discharge period.

By this valve control, the discharge gas flows continuously or intermittently during the rest period from the end of the discharge period to the start of the next preparation period, so as to, during a part or the whole of the rest period, maintain the effects of preventing the impurity gas from flowing back into the gas guide channel and remaining in the gas guide channel. It is thus more unlikely that a large amount of impurity gas will exist in the gas guide channel at the time of start of a discharge period after the rest period.

The above-mentioned plasma irradiation apparatus may comprise a concentration detection section that detects a concentration of the discharge gas or specific gas in air in a pathway portion of the gas guide channel or the gas supply channel located closer to the outlet port than the valve.

In this plasma irradiation apparatus, the concentration state of the discharge gas or the concentration state of specific gas in the pathway portion through which the discharge gas should flow (i.e. in the portion of the gas guide channel or the gas supply channel closer to the outlet port than the valve) is more accurately detected. It is thus possible to perform the control according to the current gas concentration (gas concentration at the time of detection) in such a pathway portion.

In the above-mentioned plasma irradiation apparatus, the concentration detection section may be configured to detect the concentration of the discharge gas in the pathway portion; and the discharge control section may be configured to: allow the discharge section to perform the plasma discharge when the concentration of the discharge gas detected by the concentration detection section is higher than or equal to a threshold value; and, when the concentration of the discharge gas detected by the concentration detection section is lower than the threshold value, prohibit the discharge section from performing the plasma discharge.

In this plasma irradiation apparatus, the discharge section is prohibited from performing the plasma discharge when the concentration of the discharge gas is relatively low (i.e. lower than the threshold value) in the pathway portion through which the discharge gas should flow (i.e. in the portion of the gas guide channel or the gas supply channel closer to the outlet port than the valve). The plasma discharge is thus prevented from being unstably performed in a state that the concentration of the discharge gas is too low.

In the above-mentioned plasma irradiation apparatus, the concentration detection section may be configured to detect the concentration of the specific gas in the air; and the discharge control section may be configured to increase a maximum voltage between the first and second electrodes with increase in the concentration of the specific gas detected by the concentration detection section.

The higher the concentration of the air, the lower the concentration of the discharge gas. In this plasma irradiation apparatus, the maximum voltage between the first and second electrodes is increased with decrease in the concentration of the discharge gas. Although the plasma discharge becomes difficult to perform due to decrease in the concentration of the discharge gas, such difficulty is compensated for by increasing the voltage in accordance with the degree of decrease of the discharge gas concentration.

According to still another aspect of the present invention, there is provided a plasma irradiation method using a plasma irradiation apparatus, the plasma irradiation apparatus comprising: the plasma irradiation apparatus comprising: a gas guide channel defining therein a flow path for flow of a discharge gas, with an outlet port formed at an end of the flow path; a discharge section having a first electrode, a second electrode and a dielectric layer interposed between the first electrode and the second electrode and being operable to generate plasma discharge in the gas guide channel; and a gas supply channel defining therein a path for supply of the discharge gas to the gas guide channel, the plasma irradiation method comprising:
mounting the plasma irradiation apparatus to a distal device which is movable relative to a target substance;
arranging a valve in the gas guide channel or the gas supply channel;
operating a discharge control section to control the plasma discharge of the discharge section; and
operating a valve control section to: keep the valve opened during at least a discharge period in which the discharge control section allows the discharge section to perform the plasma discharge; and open the valve during a predetermined preparation period, in which the discharge control section prohibits the discharge section from performing the plasma discharge, before the discharge period.

In this plasma irradiation method, the valve is kept opened during at least the "discharge period" in which the discharge control section allows the discharge section to perform the plasma discharge; and the valve is also opened during the "predetermined preparation method before the discharge period" in which the discharge control section prohibits the discharge section from performing the plasma discharge. In other words, the discharge gas continues flowing in the gas guide channel even during the "predetermined preparation period before the discharge period". By such valve control, any impurity gas is prevented from flowing back from the outside into the gas guide channel during the preparation period. It is thus unlikely that a large amount of impurity gas will exist in the gas guide channel at the time of start of the discharge period subsequent to the preparation period. As a consequence, the plasma discharge is prevented from being performed in a state that a large amount of impurity gas exists in the gas guide channel.

In the above-mentioned plasma irradiation method, the valve control section may be operated to open the valve at a larger opening degree during at least a partial period in the preparation period than that during the discharge period.

In this case, the discharge gas flows more vigorously into the gas guide channel during at least the partial period in the preparation period. Thus, the impurity gas is more effectively prevented from flowing into the gas guide channel during the preparation period and remaining in the gas guide channel during the preparation period.

In the above-mentioned plasma irradiation method, the valve control section may be operated to continuously or intermittently open the valve at a smaller opening degree during a rest period from an end of the discharge period to a start of the next preparation period than that during the discharge period.

In this case, the discharge gas flows continuously or intermittently during the rest period from the end of the discharge period to the start of the next preparation period, so as to, during a part or the whole of the rest period, maintain the effects of preventing the impurity gas from flowing back into the gas guide channel and remaining in the gas guide channel. It is thus more unlikely that a large amount of impurity gas will exist in the gas guide channel at the time of start of a discharge period after the rest period.

In the above-mentioned plasma irradiation method, the plasma irradiation apparatus may comprise a concentration detection section that detects a concentration of the discharge gas in a pathway portion of the gas guide channel or the gas supply channel located closer to the outlet port than the valve, and wherein the discharge control section may be operated to: allow the discharge section to perform the plasma discharge when the concentration of the discharge gas detected by the concentration detection section is higher than or equal to a threshold value; and prohibit the discharge section from performing the plasma discharge when the concentration of the discharge gas detected by the concentration detection section is lower than the threshold value.

In this plasma irradiation method, the discharge section is prohibited from performing the plasma discharge when the concentration of the discharge gas is relative low (i.e. lower than the threshold value) in the pathway portion through which the discharge gas should flow (i.e. in the portion of the gas guide channel or the gas supply channel closer to the outlet port than the valve). The plasma discharge is thus prevented from being unstably performed in a state that the concentration of the discharge gas is too low.

In the above-mentioned plasma irradiation apparatus, the plasma irradiation apparatus may comprise a concentration detection section that detects a concentration of specific gas in air in a pathway portion of the gas guide channel or the gas supply channel located closer to the outlet port than the valve, and the discharge control section may be operated to increase a maximum voltage between the first and second electrodes with increase in the concentration of the specific gas detected by the concentration detection section.

The higher the concentration of the air, the lower the concentration of the discharge gas. In this plasma irradiation method, the maximum voltage between the first and second electrodes is increased with decrease in the concentration of the discharge gas. Although the plasma discharge becomes difficult to perform due to decrease in the concentration of the discharge gas, such difficulty is compensated for by increasing the voltage in accordance with the degree of decrease of the discharge gas concentration.

### Effects of the Invention

The present invention provides the effects of preventing the plasma discharge from being performed in a state that a large amount of impurity gas exists in the gas guide channel.

### Brief Description of Drawings

FIG. 1 is a schematic view of a surgical system equipped with a plasma irradiation apparatus according to a first embodiment of the present invention and a distal device.
FIG. 2 is a schematic perspective view of a structural body of the plasma irradiation apparatus according to the first embodiment.
FIG. 3 is an exploded perspective view of the structural body of FIG. 2 in a state of being divided into three parts.
FIG. 4 is a schematic cross-sectional view of the structural body of the plasma irradiation apparatus according to the first embodiment, as taken, at a center position thereof in a third direction (width direction), along a direction perpendicular to the third direction.
FIG. 5 is a schematic cross-sectional view of the structural body of the plasma irradiation apparatus according to the first embodiment, as taken, at a center position thereof in a first direction (longitudinal direction), along a direction perpendicular to the first direction.
FIG. 6 is a schematic cross-sectional view of the structural body of the plasma irradiation apparatus according to the first embodiment, as taken, at a center position thereof in a second direction (thickness direction), along a direction perpendicular to the second direction.
FIG. 7 is a flowchart of a plasma irradiation control process executed by a plasma irradiation apparatus according to a second embodiment of the present invention.
FIG. 8 is a timing chart of the plasma irradiation control process executed by the plasma irradiation apparatus according to the second embodiment.
FIG. 9 is a schematic view showing, as a first example of the other embodiment of the present invention, an application example of a plasma irradiation apparatus to an electric knife.
FIG. 10 is a schematic view showing, as a second example of the other embodiment of the present invention, an application example of a plasma irradiation apparatus to another distal device.

### Description of Embodiments

### <First Embodiment>

### 1. Overall Structure of Surgical System

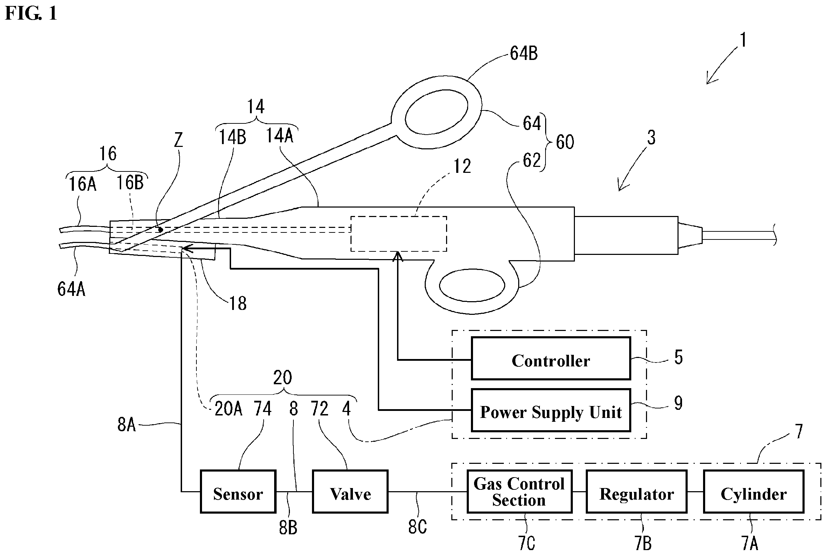

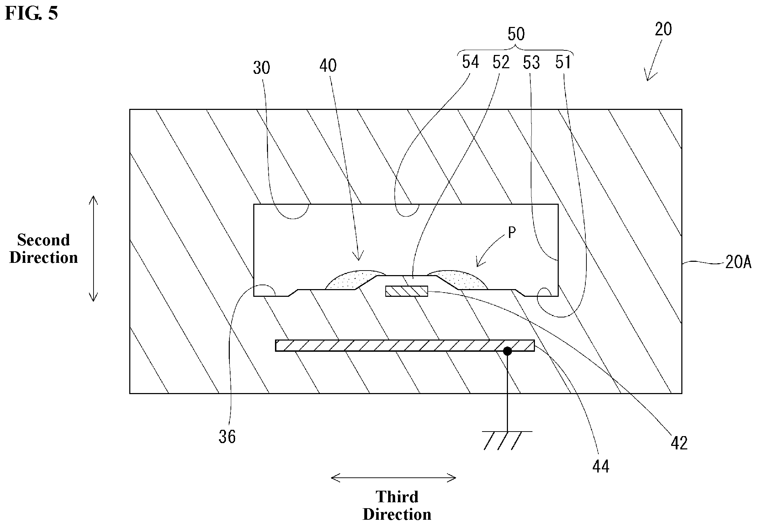

A surgical system 1 according to a first embodiment as shown in FIG. 1 is adapted as a treatment system for performing incision treatment, ablation treatment or hemostasis treatment on biological tissue set as a treatment target substance. The surgical system 1 mainly includes a distal device 3, a controller 5 that controls an ultrasonic vibration section 12 (as a drive section) of the distal device, a gas supply unit 7 that provides a gas supply to a gas guide channel 30 (see FIG. 5 etc.) inside the distal device 3, a plasma irradiation apparatus 20 and a power supply unit 9 that supplies electric power to the plasma irradiation apparatus 20.

The controller 5 is a device for executing various controls on the distal device 3. The controller 5 may be installed in a casing 14 of the distal device 3, or may be provided separately from the casing 14 and configured to send an electric signal to the casing 14 side through a flexible signal cable (not shown). The controller 5 has the functions of controlling the ultrasonic vibration section 12, receiving a detection signal from a sensor 74, controlling the power supply unit 9 etc.

The gas supply unit 7 is a device for supplying an inert gas such as helium gas, argon gas or the like (hereinafter also simply referred to as "gas"). A gas supply channel 8 is disposed between the distal device 3 and the gas supply unit 7 so that the inert gas is supplied the gas supply unit 7 to the gas guide channel 30 through the gas supply channel 8. For example, the gas supply unit 7 includes a cylinder 7A as an example of a gas supply source, a regulator 7B that decompresses high-pressure gas supplied from the cylinder 7A and a gas control section 7C that controls the decompressed gas to a desired flow rate. The gas control section 7C has e.g. a flow rate control valve for controlling the flow rate of the gas. Under the control of the controller 5 or a control device not shown, the flow rate of the discharge gas flowing through the gas supply channel 8 is controlled to the desired value.

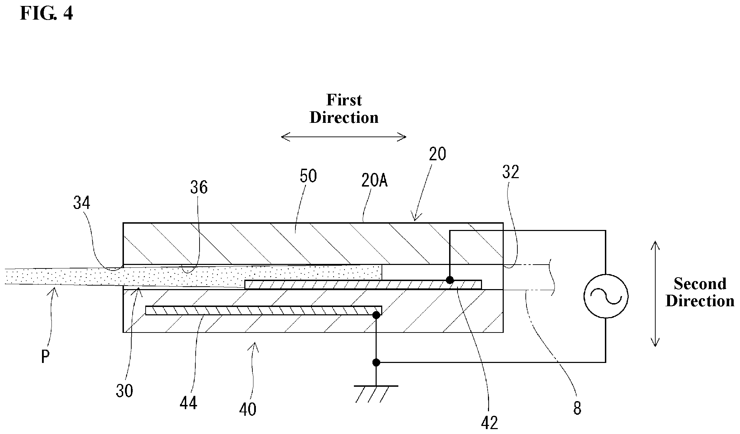

The gas supply channel 8 is a gas channel for supplying the discharge gas to the gas guide channel 30. Flexible pipes are used as part or the whole of the gas supply channel 8. The gas supply channel 8 is disposed at a location closer to the gas guide channel 30 than the gas control section 7C on a gas flow pathway from the gas supply unit 7 to an outlet port 34 (see FIG. 4), and provides a flow path for the discharge gas whose flow rate is controlled by the gas control section 7C. In the present specification, the whole of the pathway for flow of the discharge gas from the gas supply source (in the embodiment of FIG. 1, the cylinder 7A) to the gas guide channel 30 is defined as a "gas flow pathway"; and a part of the gas flow pathway from the gas control section 7C to the gas guide channel 30 is defined as the gas supply channel 8.

The power supply unit 9 is controlled by the controller 5 so as to apply a desired voltage between discharge and ground electrodes 42 and 44 of the plasma irradiation apparatus 20 according to a command signal from the controller 5. In this embodiment, the controller 5 and the power supply unit 9 are adapted to function as a discharge control section 4. More specifically, the power supply unit 9 applies an alternating voltage with a predetermined frequency between the discharge electrode 42 and the ground electrode 44 while maintaining the ground electrode 44 at a ground potential. The power supply unit 9 can adopt any of various known circuits capable of generating a high voltage (e.g. a high voltage having an amplitude of 0.5 kV to 10 kV) with a high frequency (of e.g. about 20 kHz to 300 kHz). The frequency of the high voltage generated by the power supply unit 9 may be fixed at a constant value or may be varied. Further, the voltage applied between the discharge electrode 42 and the ground electrode 44 by the power supply unit 9 is preferably a periodically changing voltage such as an alternating voltage of sinusoidal waveform or an alternating voltage of nonsinusoidal waveform (e.g. rectangular waveform, triangular waveform etc.).

Although the surgical system is exemplified in which the power supply unit 9 for generating an alternating voltage is disposed outside of the distal device 3 in the embodiment of FIG. 1, a power supply circuit for generating an alternating voltage may be disposed inside of the distal device 3 (e.g. in the casing 14 or in the plasma irradiation apparatus 20).

The distal device 3 is a device held and used by an operator who carries out surgical operator in such a manner that the operator moves the distal device 3 relative to the treatment target substance (such as biological tissue). The distal device 3 mainly includes the casing 14, an acting member 16, the plasma irradiation apparatus 20, the ultrasonic vibration section 12 and the like. The casing 14, the acting member 16, at least a part of the plasma irradiation apparatus 20 (such as a plasma generator 20A) and the ultrasonic vibration section 12 are integrated into one as a grip unit (hand grip unit) gripped by the operator. The inert gas and electric power are supplied to this unit through the flexible members.

The casing 14 is formed in a cylindrical shape extending in a predetermined direction, and generally has a base portion 14A and a cylindrical extending portion 14B formed integral with the base portion 14A and extending in the predetermined direction. The ultrasonic vibration section 12 is accommodated in the base portion 14A, whereas the plasma generator 20A as a part of the plasma irradiation apparatus 20 is fixed to or integrated with the extending portion 14B.

The ultrasonic vibration section 12 is configured as a known ultrasonic vibrator. When a predetermined electric signal is sent to the ultrasonic vibration section 12 by the controller 5, the ultrasonic vibration section 12 is driven to generate and transmit ultrasonic vibration to the shaft-shaped acting member 16. The ultrasonic vibration section 12 corresponds to one example of the drive section, and drives the acting member 16 in such a manner that incision action, ablation action or thermocoagulation hemostasis action takes place on the biological tissue in the vicinity of an acting portion 16A.

The acting member 16 is a member whose front end part acts as a stationary blade on the biological tissue. The acting member 16 corresponds to an example of a vibratory member to which ultrasonic vibration generated by the ultrasonic vibration section 12 is transmitted. The acting member 16 has: the acting portion 16A formed on the front end part thereof; and a shaft portion 16B that transmits the vibration from the ultrasonic vibration section 12 to the acting portion 16A. The acting member 16 is hence adapted to cause vibration of the acting portion 16A by transmission of the ultrasonic vibration from the ultrasonic vibration unit 12 to the acting portion 16A through the shaft portion 16B. Accordingly, the acting member 16 is operated to perform incision action, ablation action or hemostasis action on the biological tissue by causing vibration of the acting portion 16A in a state that the acting portion 16A is in close proximity to or in contact with the biological tissue.

The distal device 3 also includes a grip part 60 gripped and used by the operator. The grip part 60 is structured as a movable member displacement mechanism. Any known moving mechanism is employed as the movable member displacement mechanism. The grip part 60 has: a stationary grip portion 62 integrated with the casing 14 by being fixed to the base portion 14A of the casing 14; and a movable member 64 formed in a shaft shape and attached movably relative to the stationary grip portion 62. A movable blade 64A is provided on one end part of the movable member 64, whereas a movable grip portion 64B is provided on the other end part of the movable member 64. In the grip part 60, the shaft-shaped movable member 64 is pivotable on a pivot axis Z which is located near a front end of the extending portion 14B. When the grip part 60 is operated to bring the movable grip portion 64B closer to the stationary grip portion 62, the movable member 64 pivots in such a manner that the movable blade 64A moves toward the acting portion 16A (stationary blade) of the acting member 16. When the grip part 60 is operated to bring the movable grip portion 64B away from the stationary grip portion 62, the movable member 64 pivots in such a manner that the movable blade 64A moves away from the acting portion 16A (stationary blade).

The above-configured distal device 3 is used to perform incision treatment, ablation treatment or hemostasis treatment on the biological tissue by application of ultrasonic vibration. For example, incision treatment can be performed on the biological tissue by transmitting the ultrasonic vibration to the acting portion 16A in a state that the biological tissue is nipped between the acting portion 16A (stationary blade) and the movable blade 64A. Hemostasis treatment can be performed on the biological tissue through the application of frictional heat by transmitting the ultrasonic vibration to the acting portion 16A in a state that the acting portion 16A is brought into contact with the biological tissue. Further, ablation treatment can performed on the biological tissue by nipping the biological tissue between the acting portion 16A and the movable blade 64A while transmitting or not transmitting the ultrasonic vibration to the acting member 16. The distal device 3 is also used to, in combination with performing incision treatment, ablation treatment or thermocoagulation hemostasis treatment by application of ultrasonic vibration as described above, perform minimally invasive hemostasis treatment on the biological tissue by irradiation with low-temperature plasma from the plasma irradiation apparatus 20 as will be described later.

### 2. Basic Configuration of Plasma Irradiation Apparatus

Next, the configuration of the plasma irradiation apparatus 20 will be described in detail below.

As shown in FIG. 1, the plasma irradiation apparatus 20 is installed as a part of the distal device 3 and is configured to generate dielectric barrier discharge within the distal device 3. In the embodiment of FIG. 1, the plasma generator 20A as a part of the plasma irradiation apparatus 20 is fixed to the casing 14 in a state of being held by a holding part 18. The holding part 18, which holds and accommodates therein the plasma generator 20A, may be in the form of a casing member having a casing body made of a metal material and covered with a resin coating or a casing member having a casing body made of a resin material and covered with a metal plating. The use of such a holding part contributes to suppression of unintentional discharge or electrical leakage from the plasma generator 20A. Low-temperature plasma generated inside the plasma generator 20A is emitted to the vicinity of the acting portion 16A which is provided on the front end part of the acting member 16.

As shown in FIG. 1, the plasma irradiation apparatus 20 mainly includes the plasma generator 20A, the gas supply channel 8, the sensor 74, a valve 72 and the discharge control section 4.

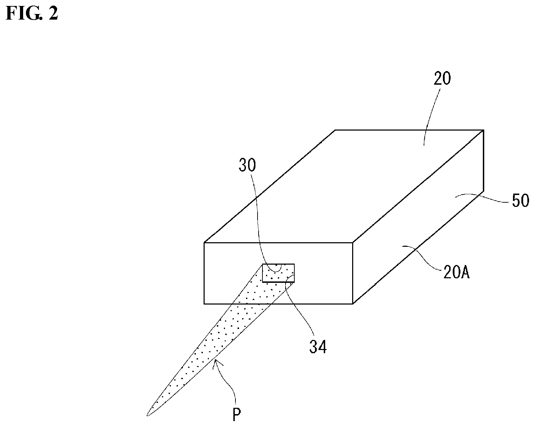

The plasma generator 20A has a structural body of predetermined three-dimensional shape (such as plate shape or rectangular parallelepiped shape) as shown in FIG. 2. The outlet port 34 is formed in a longitudinal end of the plasma generator 20A such that the low-temperature plasma P is emitted from the outlet port 34.

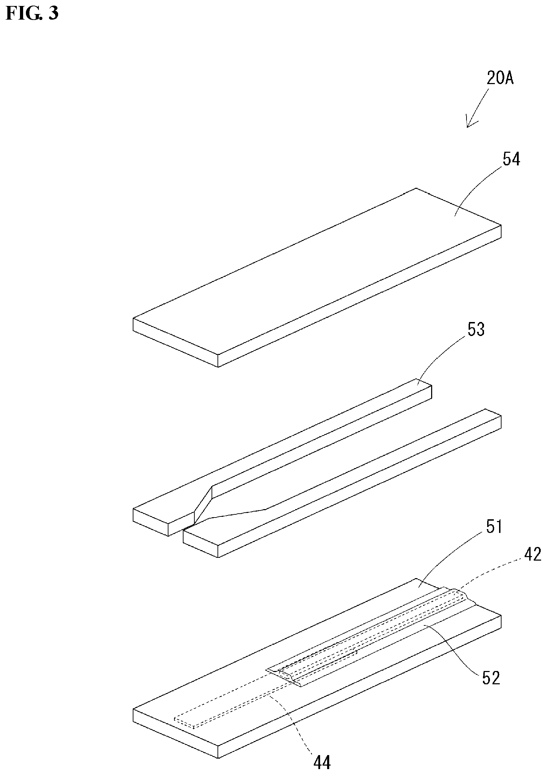

As schematically shown in FIG. 3, the plasma generator 20A includes: a third dielectric layer 53 located at a center thereof in a thickness direction; a fourth dielectric layer 54 located on one side of the third dielectric layer 53 in the thickness direction; and first and second dielectric layers 51 and 52 located on the other side of the third dielectric layer 53 in the thickness direction. The discharge electrode 42 and the ground electrode 44 are embedded in the dielectric region constituted by the first and second dielectric layers 51 and 52. In this embodiment, the discharge electrode 42 corresponds to an example of a first electrode; and the ground electrode 44 corresponds to an example of a second electrode. In FIG. 3, the plasma generator 20A is schematically shown in exploded perspective view in a state of being divided into three parts. In practice, however, the first dielectric layer 51, the second dielectric layer 52, the third dielectric layer 53 and the fourth dielectric layer 54 are provided as portions of an integral dielectric member 50 (see FIG. 4).

The plasma generator 20A is generally provided with the gas guide channel 30 and a creeping discharge section 40 as shown in FIG. 4.

The gas guide channel 30 has an inlet port 32 for gas introduction, outlet port 34 for gas discharge and a flow path 36 that extends between the inlet port 32 and the outlet port 34. In other words, the gas guide channel 30 is a gas channel for introducing the inert gas supplied by the gas supply unit 7, which is disposed outside the distal device 3, from the inlet port 32 and guiding the introduced gas to the outlet port 34 through an inner space of the flow path 36. In FIG. 4, the gas supply channel 8 for guiding the inert gas from the gas supply unit 7 to the inlet port 32 is schematically shown by a two-dot chain line. The outlet port 34 of the gas guide channel 30 is arranged at a position adjacent to the acting portion 16A and is directed toward the acting portion 16A such that the acting portion 16Ais located on an extension of the inner space of the flow path 36. The gas guide channel 30 is thus adapted to emit the gas from the outlet port 34 toward the acting portion 16A. Consequently, the gas guide channel 30 functions to emit the low-temperature plasma P together with the gas from the outlet port 34 to the acting portion 16A.

As shown in FIG. 4, a direction along which the gas guide channel 30 extends in the plasma irradiation apparatus 20 is defined as a first direction. Among directions perpendicular to the first direction, a thickness direction of the dielectric member 50 is defined as a second direction; and a direction perpendicular to the first and second directions is defined as a third direction (see FIG. 5). In FIG. 4, a longitudinal direction of the plasma generator 20A in which the dielectric member 50, the discharge electrode 42 and the ground electrode 44 are integrated together corresponds to the first direction. When the plasma generator 20A is viewed in cross section along a plane perpendicular to the first direction, a short dimension direction of the cross section of the plasma generator 20A corresponds to the second direction; and a long dimension direction of the cross section of the plasma generator 20A corresponds to the third direction. In this embodiment, the second direction is a width direction of the plasma generator 20A; and the third direction is a height direction or thickness direction of the plasma generator 20A. In the following description, the outlet port 34 side in the first direction is referred to as a front side of the plasma generator 20A; and the inlet port 32 side in the first direction is referred to as a rear side of the plasma generator 20A.

The creeping discharge section 40 is constituted by the first dielectric layer 51, the discharge electrode 42 and the ground electrode 44. The discharge electrode 42 and the ground electrode 44 are opposed to each other with the first dielectric layer 51 interposed therebetween. The creeping discharge section 40 corresponds to an example of a discharge section, and functions to develop, in the gas guide channel 30, an electric field based on a potential difference between the discharge electrode 42 and the ground electrode 44 and thereby generate low-temperature plasma discharge as a result of creeping discharge.

More specifically, the creeping discharge section 40 is disposed such that one of the discharge electrode 42 and the ground electrode 44 faces the flow path 36 directly or via another member and is configured to generate creeping discharge by the application of a periodically changing voltage to the discharge electrode 42. The configuration in which "one of the discharge electrode 42 and the ground electrode 44 faces the flow path 36 directly" corresponds to the case where one of the discharge electrode 42 and the ground electrode 44 is exposed inside the inner space of the flow path 36 and constitutes a part of the inner wall of the flow path. The configuration in which "one of the discharge electrode 42 and the ground electrode 44 faces the flow path 36 via another member" corresponds to the case where the one of the discharge electrode 42 and the ground electrode 44 is located at a position near the flow path 36 and is partially or wholly covered by another member. In the latter configuration, the another member constitutes a part of the inner wall of the flow path; and a main surface of the one of the electrodes faces the flow path 36. Although the discharge electrode 42 corresponds to the one of the electrodes and faces the flow path 36 via the another member in the embodiment of FIGS. 4 and 5, the second dielectric layer 52 corresponding to one example of the another member is omitted from illustration in FIG. 4.

As shown in FIG. 5, the creeping discharge section 40 is disposed such that the discharge electrode 42 faces the flow path 36 via a part of the dielectric member 50 (i.e. via the second dielectric layer 52). The ground electrode 44 is situated on a side opposite from the flow path 36 with respect to the discharge electrode 42 and is thus located further apart from the flow path 36 than the discharge electrode 42. The creeping discharge section 40 generates low-temperature plasma as a result of creep discharge by the application of a periodically changing voltage to the discharge electrode 42 while maintaining the potential of the ground electrode 44 at a constant reference potential (e.g. a ground potential of 0 V).

As shown in FIG. 5, the dielectric member 50 is provided with the first, second, third and fourth dielectric layers 51, 52, 53 and 54 and formed in a hollow shape as a whole. The first dielectric layer 51 corresponds to an example of a "dielectric part having a portion thereof interposed between the discharge electrode 42 (first electrode) and the ground electrode 44 (second electrode)". The first dielectric layer 51 is arranged on one side of the flow path 36 in the second direction (thickness direction), with the ground electrode 44 embedded in the first dielectric layer 51. The second dielectric layer 52 is made of a ceramic material as a ceramic protective layer and arranged at a position closer to the space of the flow path than the first dielectric layer 51 so as to cover the discharge electrode 42. These first and second dielectric layers 51 and 52 constitute an inner wall of the flow path 36 on one side in the second direction. The fourth dielectric layer 54 is arranged on the other side of the flow path 36 in the second direction (thickness direction), and constitutes an inner wall of the flow path 36 on the other side in the second direction. The third dielectric layer 53 is arranged between the first dielectric layer 51 and the fourth dielectric layer 54 in the second direction, and constitutes side walls of the flow path 36 on one and the other sides in the third direction. The flow path 36 is therefore defined by the first, second, third and fourth dielectric layers 51, 52, 53 and 54. As the materials of the first, second, third and fourth dielectric layers 51, 52, 53 and 54, there can suitably be used a ceramic material such as alumina, a glass material or a resin material. Herein, the use of alumina with high mechanical strength as the material of the dielectric layer facilitates downsizing of the creeping discharge section 40.

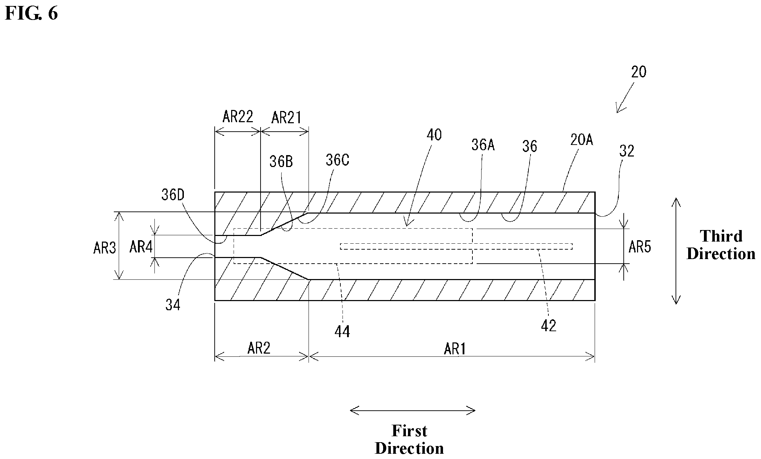

As shown in FIG. 6, the flow path 36 includes: a first flow passage 36A whose space is surrounded at both sides in the second direction and at both sides in the third direction and continues and extends in the first direction; and a second flow passage 36B disposed on a downstream side of the first flow passage 36A. The first flow passage 36A is arranged in a first area AR1 of the plasma generator 20A in the first direction, whereas the second flow passage 36B is arranged in a second area AR2 of the plasma generator 20A in the first direction. In FIG. 6, the range of arrangement of the first flow passage 36A in the first direction is indicated as the first area AR1; and the range of arrangement of the second flow passage 36B in the first direction is indicated as the second area AR2.

The first flow passage 36A has an inner circumferential wall surface rectangular in shape (see FIG. 5) when viewed in cross section in a direction perpendicular to the first direction. As shown in FIG. 6, a width of the inner circumferential wall surface of the first flow passage 36A is made constant throughout the entire first area AR1 in the first direction; and a height of the inner circumferential wall surface of the first flow passage 36A is made constant throughout the entire first area AR1 in the first direction.

As shown in FIG. 6, the second flow passage 36B is located closer to the outlet port 34 (downstream side) than the first flow passage 36Ain the first direction and is made narrower in width than the first flow passage 36A. The second flow passage 36 has a reduced width passage portion 36C and a constant width passage portion 36D. The reduced width passage portion 36C is situated within a partial area AR21 in the second area AR2 in the first direction. A width of the inner circumferential wall surface of the reduced width passage portion 36C gradually decreases toward the outlet port 34. A height of the inner circumferential wall surface of the reduced width passage portion 36C is made constant throughout the entire partial area AR21. The constant width passage portion 36D is situated within a partial area AR22 in the second area AR2 in the first direction. A width and height of the inner circumferential wall surface of the constant width passage portion 36D are made constant throughout the entire partial area AR22.

The ground electrode 44 extends linearly in the first direction so as to lie along the flow path 36. For example, the ground electrode 44 is made constant in width and thickness and is disposed within a predetermined area in the first direction. The ground electrode 44 is positioned such that a front end portion of the ground electrode 44 is located closer to the outlet port 34 than the discharge electrode 43. More specifically, a part of the ground electrode 44 is situated in the arrangement area AR2 of the second flow passage 36B in the first direction. In the embodiment of FIG. 6, a front end of the ground electrode 44 is located frontward of a front end of the reduced width passage portion 36C (i.e. a rear end of the constant width passage portion 36D). In other words, a part of the ground electrode 44 is situated in the arrangement area AR22 of the constant width passage portion 36D in the first direction. Further, a rear end of the ground electrode 44 is located rearward of a front end of the first flow passage 36A and is located rearward of a front end of the discharge electrode 42 and frontward of a rear end of the discharge electrode 42. At least a part of the ground electrode 44 (in FIG. 6, the whole of the ground electrode 44) is situated in the arrangement area AR3 of the first flow passage 36A in the third direction. More specifically, at least a part of the ground electrode 44 (in FIG. 6, a part of the ground electrode 44) is situated in the arrangement area AR4 of the constant width passage portion 36D in the third direction and is situated in the formation area of the outlet port 34 in the third direction.

The discharge electrode 42 extends linearly in the first direction so as to lie along the flow path 36. For example, the discharge electrode 42 is made constant in width and thickness and is disposed within a predetermined area in the first direction. More specifically, the discharge electrode 42 is situated only in the arrangement area of the first flow passage 36A in the first direction. That is to say, the discharge electrode 42 is situated only in the first area AR1 among the first and second areas AR1 and AR2. A front end of the discharge electrode 42 is located rearward of a front end of the first flow passage 36A. A rear end of the discharge electrode 42 is located frontward of a rear end of the first flow passage 36A. Further, the width (length in the third direction) of the discharge electrode 42 is made narrower than the width (length in the third direction) of the ground electrode 44. In the embodiment of FIG. 6, the discharge electrode 42 is situated in the arrangement area AR3 of the first flow passage 36A in the third direction. More specifically, the discharge electrode 42 is situated in the arrangement area AR4 of the constant width passage portion 36D in the third direction and is situated in the formation area of the outlet port 34 in the third direction. To be more specific, the discharge electrode 42 is situated in the arrangement area AR5 of the ground electrode 44 in the third direction. An edge of the discharge electrode 42 on one side in the third direction is located closer, than an edge of the ground electrode 44 on one side in the third direction, to the other side in the third direction. An edge of the discharge electrode 42 on the other side in the third direction is located closer to one side in the third direction than an edge of the ground electrode 44 on the other side in the third direction.

The sensor 74 (see FIG. 1) is adapted to detect the concentration of measurement target gas in the gas guide channel 30 or in a portion of the gas flow pathway located closer to the outlet port than the valve 72. The sensor 74 may have the function of detecting the concentration of the discharge gas or may have the function of detecting the concentration of specific gas (such as oxygen) in air. In the case where the discharge gas contains a plurality of gas components, the sensor 74 may have the function of detecting any specific gas component contained in the discharge gas.

The above-configured plasma irradiation apparatus 20 is supplied with an inert gas such that the inert gas flows through the inner space of the flow path 36. Then, an alternating voltage with a predetermined frequency is applied between the discharge electrode 42 and the ground electrode 44 by the power supply unit 9 so as to, for example, while maintaining the ground electrode 44 at a ground potential, oscillate the potential of the discharge electrode 42 within the range between potential values of +A (V) and -A (V) which are respectively higher and lower by a certain degree than the potential of the ground electrode 44. Herein, the value "A" is a positive value. With the application of such an alternating voltage, there occurs a change of electric field between the electrodes in a state that a barrier is formed by the dielectric member 50. As a result, dielectric barrier discharge (more specifically, creeping discharge) is generated in the inner space of the gas guide channel 30. As the inert gas flows to the outlet port 34 in the gas guide channel 30, low-temperature plasma resulting from the creeping discharge is emitted with the gas from the outlet port 34 to the acting portion 16A. Accordingly, when the operator directs the acting portion 16A of the distal device 3 toward e.g. a bleeding site and operates the plasma irradiation apparatus 20, low-temperature plasma is emitted to the bleeding site whereby coagulation of blood can be caused for hemostasis of the bleeding site.

### 3. Configuration of Valve 72

The configuration of the valve 72 as one feature of the present invention will be next described below.

In the plasma irradiation apparatus 20, the valve 72 is arranged at some point in the gas supply channel 8 as shown in FIG. 1.

In this embodiment, the valve 72 is configured as a check valve. In the case where the valve 72 is configured as a check valve, the check valve can be of any known type capable of preventing a backflow of gas. Various types of check valves, such as those of diaphragm type, ball type, O-ring type, spring disc type, swing type etc., are usable.

The valve 72 is mounted to the gas supply channel 8 so as to allow a flow of the inert gas in a direction toward the outlet port 34 but prevent a flow of the gas in a reverse direction. More specifically, assuming the gas control section 7C side as the upstream side and the outlet port 34 side as the downstream side, the valve 72 allows a flow of the discharge gas from the upstream side to the downstream side (i.e. a flow of the discharge gas from the gas control section 7C side to the outlet port 34 side) and interrupts a flow of the discharge gas from the downstream side to the upstream side (i.e. a flow of the discharge gas from the outlet port 34 side to the gas control section 7C side). There thus occurs no flow of the gas from the downstream side to the upstream side through the valve 72.

In FIG. 1, a portion of the gas supply channel 8 between the sensor 74 and the gas guide channel 30 is denoted by reference numeral 8A; a portion of the gas supply channel 8 between the sensor 74 and the valve 72 is denoted by reference numeral 8B; and a portion of the gas supply channel 8 between the valve 72 and the gas control section 7C is denoted by reference numeral 8C. Each of these supply channel portions 8A, 8B and 8C can be in the form of a pipe without another member mounted thereto or in the form of a pipe with another member (such as sensor, valve etc.) mounted thereto.

Next, the effects of the above-described configuration will be discussed below.

In the plasma irradiation apparatus 20, the valve 72 functioning as a check valve is arranged in the gas flow channel 8 (more specifically, in a portion of the gas supply channel closer to the gas guide channel 30 than the gas control section 7C for flow rate control of the discharge gas). As the valve 82 (check valve) is adapted to allow a flow of the discharge gas in the direction toward the outlet port 34 and prevent a flow of the gas in the reverse direction, a large amount of impurity gas (such as air) different from the discharge gas is prevented from flowing into piping upstream of the valve 72 (check valve) (i.e. piping closer to the gas control section than the check valve). As a result, the occurrence of unstable discharge due to the entry of a large amount of impurity gas is suppressed.

### <Second Embodiment>

A second embodiment will be next described below.

A plasma irradiation apparatus 20 according to the second embodiment is different from that according to the first embodiment in that: a valve 72 having a different function from that of the first embodiment is provided; and an additional function is imparted to the controller 5. Hence, a detailed description of the same parts and portions as those of the first embodiment will be omitted herefrom. The contents of the first embodiment described above in "1. Overall Structure of Surgical System" and "Basic Configuration of Plasma Irradiation Apparatus" are applied to the second embodiment. Since the same structure and configuration as those of the first embodiment shown in FIGS. 1 to 6 are applied to the second embodiment, the following description of the second embodiment will be made with reference to FIGS. 1 to 6.

In the plasma irradiation apparatus 20 of the second embodiment, the valve 72 is arranged as a flow control valve in the gas flow pathway. This valve 72 has the function of controlling the flow rate of the discharge gas according to its opening degree.

As in the case of the first embodiment, the power supply unit 9 and the controller 5 function as the discharge control section 4 in the second embodiment. The discharge control section 4 controls the creeping discharge section 40 (discharge section) to generate plasma discharge by controlling a voltage applied between the discharge electrode 42 (first electrode) and the ground electrode 44 (second electrode).

Further, the controller 5 has the additional function of controlling opening and closing of the valve 72 (valve). In other words, the controller 5 corresponds to an example of a valve control section in the plasma irradiation apparatus 20 of the second embodiment.

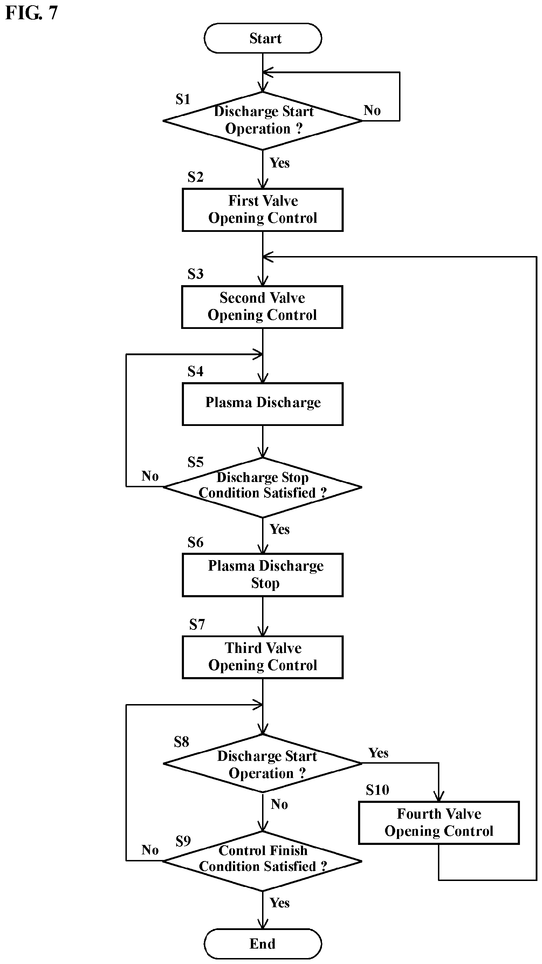

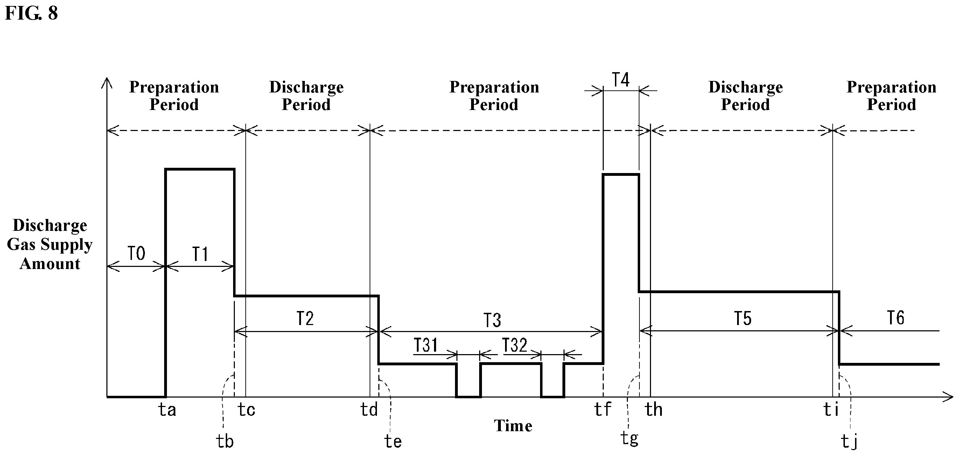

Next, a plasma discharge control process executed by the plasma irradiation apparatus 20 of the second embodiment will be described below with reference to FIGS. 7 and 8. FIG. 8 is a timing chart of one example of the plasma discharge control process executed according to a flowchart of FIG. 7.

In the second embodiment, a plasma irradiation method is embodied as shown in the flowchart of FIG. 7, with the plasma irradiation apparatus 20 being mounted to the distal device 3, which is movable relative to the target substance (such as biological tissue), and the valve 72 (capable of controlling the flow rate of the discharge gas according to its opening degree) being mounted to the gas flow pathway. In this plasma irradiation method, the discharge control section 4 controls plasma discharge of the creeping discharge section 40 (discharge section). The controller 5 (valve control section) keeps the valve 72 (valve) opened at least during a discharge period in which the discharge control section 4 allows the creeping discharge section 40 (discharge section) to perform plasma discharge. Furthermore, the controller 5 opens the valve 72 (valve) even during a "predetermined preparation period" which is prior to the discharge period and in which the discharge control section 4 prohibits the creeping discharge section 40 (discharge section) from performing plasma discharge.

Herein, a program for execution of the plasma discharge control process of FIG. 7 is stored in e.g. a memory of the controller 5 and executed by a control circuit (such as CPU) of the controller 5.

The controller 5 initiates the plasma discharge control process of FIG. 7 upon satisfaction of a predetermined control start condition. The predetermined control start condition can be a condition in which the controller 5 is powered on or a condition in which a predetermined control start operation is made by an operation section (not shown).

After the start of the control process of FIG. 7, the controller 5 judges in step S1 whether there is made a discharge start operation. The discharge start operation is a predetermined operation for instructing a start of the plasma discharge. The discharge start operation can be an operation made on an operation section (such as operation button) of the casing 14 etc. as an instruction for starting plasma discharge or any other predetermined operation. The plasma discharge control process of FIG. 7 is in a standby state up until the discharge start operation is made after the start of the control process of FIG. 7. In the example of FIG. 8, the period T0 up until time ta corresponds to a duration of the standby state.

Upon judging in step S1 that the discharge start operation is made, the controller 5 executes first valve opening control in step S2. The first valve opening control is to open the valve 72 (valve) at a first opening degree during a certain time period T1. The first opening degree is set to a degree larger than the opening degree (second opening degree) of the valve 72 during the discharge period. Hence, the rate of flow of the discharge gas in the gas guide channel 30 during the period T1 of the first valve opening control is set higher than the rate of flow of the discharge gas in the gas guide channel 30 during the discharge period. In the example of FIG. 8, the first valve opening control is started from time ta and stopped at time tb shortly before time tc of start of the discharge period.

After the execution of the first valve opening control in step S2, the controller 5 executes second valve opening control in step S3. The second valve opening control is to open the valve 72 (valve) at the second opening degree, which should be set for the discharge period, and maintaining such a valve opening state. In the example of FIG. 8, the opening degree of the valve 72 is set to the first opening degree during the period from time ta to time tb and set to the second opening degree during the period from time tb to time te. That is, the opening degree of the valve 72 is switched at time tb.

After the start of the second valve opening control in step S3, the controller 5 executes plasma discharge control in step S4. Upon starting the plasma discharge control in step S4, the controller 5 controls the creeping discharge section 40 to perform plasma discharge by the application of an alternating voltage with a predetermined frequency between the discharge electrode 42 and the ground electrode 44. After the start of the plasma discharge control in step S4, the controller 5 judges in step S5 whether a discharge stop condition is satisfied. When the discharge stop condition is not satisfied, the process goes back to step S4. Then, the controller 5 repeats the processing of steps S4 and S5 until satisfaction of the discharge stop condition. Upon judging that the discharge stop condition is satisfied, by contrast, the controller 5 goes to step S6 and then stops the plasma discharge in step S6. After the stop of the plasma discharge in step S6, the controller 5 switches from the second valve opening control to third valve opening control in step S7. The discharge stop condition can be a condition in which a predetermined stop operation is made on the operation section of the casing 14 etc. or any time condition (e.g. a condition in which a predetermined time period has elapsed from the start of the plasma discharge).

In the example of FIG. 8, the controller 5 starts the second valve opening control of step S3 at time tb and starts the plasma discharge control of step S4 at time tc. Since the plasma discharge control is started at time tc after the second valve opening control is started at time tb, the plasma discharge is performed in a state that the opening degree of the valve 72 has been switched to the second opening degree. Herein, a period before time tc corresponds to the "preparation period" during which the plasma discharge is not performed.

The controller 5 continues the second valve opening control and the plasma discharge control after the plasma discharge is started at time tc and until the discharge stop condition is satisfied (i.e. Yes judgment is made in step S5), and then, stops the plasma discharge at time td where the discharge stop condition is satisfied. In other words, a period from time tc to time td corresponds to the "discharge period" in which the plasma discharge is performed. After the plasma discharge is stopped at time td, the second valve opening control is switched to the third valve opening control at time te. The opening degree of the valve 72 is thus maintained at the second opening degree during the whole of the "discharge period". Consequently, the rate of flow of the discharge gas in the gas guide channel 30 is maintained at a level corresponding to the second opening degree during the whole of the "discharge period".

After the start of the third valve opening control in step S7, the controller 5 intermittently opens the valve 72 (valve). More specifically, the controller 5 causes opening and closing of the valve 72 so as to alternatingly repeat a first time duration during which the valve 72 is opened at a third opening degree smaller than the second opening degree and a second time duration during which the valve 72 is closed. By such valve control, the time duration during which the discharge gas flows in the gas guide channel 30 at a lower flow rate than that during the "discharge period" and the time duration during which the discharge gas does not flow in the gas guide channel 30 are alternatingly repeated during the period of the third valve opening control. In the example of FIG. 8, the controller 5 executes the third valve opening control during the period T3 from time t3 to time tf such that the supply of the discharge gas is stopped during predetermined periods T31 and T32 in the third valve opening control period.

After step S7, the controller 5 judges in step S8 whether there is made a discharge start operation. This discharge start operation can be the same as or different from the discharge start operation of step S1. Upon judging in step S8 that the discharge start operation is made, the controller 5 judges in step S9 whether a control finish condition is satisfied. The process goes back to step S8 upon judging in step S9 that the control completion condition is not satisfied. Then, the controller 5 repeats the processing of steps S8 and S9 until the discharge start operation is made or until the control completion condition is satisfied. The third valve opening control, started in step S7 after step S6, is continued until a Yes judgement is made in step S8 or S9. The control finish condition can be a condition in which the controller 5 is turned into a power-off state (e.g. a state where no power is supplied to the controller 5) or a condition in which a predetermined control finish operation is made on the operation section.

Upon judging in step S8 that the discharge start operation is made, the controller 5 starts fourth valve opening control in step S10. The fourth valve opening control is to open the valve 72 (valve) at a fourth opening degree during a certain time period T4. The fourth opening degree is set to a degree larger than the opening degree (second opening degree) of the valve 72 during the discharge period, and can be the same as or different from the first opening degree. In the example of FIG. 8, the first opening degree and the fourth opening degree are set to the same degree. Further, the length of the certain time period T4 can be the same as or different from the length of the certain time period T1. In the example of FIG. 8, the certain time period T4 is set shorter in length than the certain time period T1. The rate of flow of the discharge gas in the gas guide channel 30 during the fourth valve opening control is set higher than the rate of flow of the discharge gas in the gas guide channel 30 during the discharge period. In the example of FIG. 8, the fourth valve opening control is started from time tf and stopped at time tg shortly before time th of start of the next discharge period. After the execution of the fourth valve opening control, the process goes back to step S3. Then, the controller 5 performs the processing of step S3 and subsequent steps. In the example of FIG. 8, there is shown a case where the control goes back to step S3 so that the processing of step S3 is started at time tg. In other words, the fourth valve opening control is switched to the second valve opening control at time tg.

The effects of the above-described configuration will be next described below.

In the plasma irradiation apparatus 20, the valve 72 (valve) is kept open during at least the "discharge period" in which the discharge control section 4 controls the creeping discharge section 40 (discharge section) to perform plasma discharge. The valve 72 (valve) is also opened during the "predetermined preparation period before the discharge period" in which the discharge control section 4 controls the creeping discharge section 40 (discharge section) not to perform plasma discharge. In other words, the discharge gas is kept flowing in the gas channel 30 even during the preparation period before the discharge period. By this control, any impurity gas flowing back from the outside into the gas guide channel 30 is discharged out during the preparation period. It is thus unlikely that a large amount of impurity gas will exist in the gas guide channel 30 at the time of start of the discharge period subsequent to the preparation period. As a consequence, the plasma discharge is prevented from being performed in a state that a large amount of impurity gas exists in the gas guide channel 30.

The controller 5 (valve control section) functions to open the valve 72 (valve) at a larger opening degree during at least a partial period in the preparation period than that during the discharge period so that the discharge gas flows in the gas guide channel 30 more vigorously during at least the partial period in the preparation period. Thus, the impurity gas in the gas guide channel 30 is discharged out more effectively during the preparation period.

Further, the controller 5 (valve control section) functions to intermittently open the valve 72 (valve) at a smaller opening degree during a rest period from the end of the discharge period to the start of the next preparation period (in FIG. 8, the period from time td to time tf) than that during the discharge period. In other words, the plasma irradiation apparatus allows intermittent flow of the discharge gas during the rest period from the end of the discharge period to the start of the next preparation period, so as to, during a part or the whole of the rest period, maintain the effects of preventing the impurity gas from flowing back into the gas guide channel 30 and remaining in the gas guide channel 30. It is thus more unlikely that a large amount of impurity gas will exist in the gas guide channel at the time of start of the discharge period after the rest period.

In the plasma irradiation apparatus 20, the sensor 74 serves as an example of a concentration detection section to detect the concentration of the discharge gas or specific gas in air in a portion of the gas flow pathway closer to the outlet port 34 than the valve 72 (valve). The concentration state of the discharge gas or specific gas in the pathway portion through which the discharge gas should flow (i.e. in the portion of the gas flow pathway closer to the outlet port 34 than the valve 72 (valve)) is more accurately detected. It is thus possible to execute the control process according to the current gas concentration (gas concentration at the time of detection) in such a pathway portion. This function may be imparted to the plasma irradiation apparatus 20 of the first embodiment.

In the case where the sensor 74 has the function of detecting the concentration of the discharge gas, the discharge control section 4 may be configured to allow the creeping discharge section 40 (discharge section) to perform plasma discharge when the concentration of the discharge gas detected by the sensor 74 (concentration detection section) is higher than or equal to a threshold value. The discharge control section 4 may be configured to, when the concentration of the discharge gas detected by the sensor 74 (concentration detection section) is lower than the threshold value, prohibit the creeping discharge section 40 (discharge section) from performing plasma discharge. With such configuration, the creeping discharge section 40 (discharge section) is prohibited from performing plasma discharge when the concentration of the discharge gas is relative low (i.e. lower than the threshold value) in the pathway portion through which the discharge gas should flow (i.e. in the portion of the gas flow pathway closer to the outlet port 34 than the valve 72 (valve)). The plasma discharge is thus prevented from being unstably performed in a state that the concentration of the discharge gas is too low.

More specifically, a "state in which the concentration of the discharge gas detected by the sensor 74 (concentration detection section) becomes lower than the threshold" is adoptable as one discharge stop condition in the control process of FIG. 7. In such a case, the controller may be configured to judge that the discharge stop condition is satisfied when the concentration of the discharge gas detected by the sensor 74 (concentration detection section) becomes lower than the threshold value during a period from the time at which the processing of step S4 is first started to the time at which any discharge stop condition is next satisfied (i.e. next Yes judgement is made in step S5), and then, stop the plasma discharge in step S6. Alternatively, the controller may be configured to: stop the plasma discharge temporarily when the concentration of the discharge gas detected by the sensor 74 (concentration detection section) becomes lower than the threshold value during a period from the time at which the processing of step S4 is first started to the time at which any discharge stop condition is next satisfied (i.e. next Yes judgement is made in step S5); and restart the plasma discharge when the concentration of the discharge gas detected by the sensor 74 (concentration detection section) becomes higher than or equal to the threshold value during a period up until the discharge stop condition is next satisfied.

This function may be imparted to the plasma irradiation apparatus 20 of the first embodiment.

In the case where the sensor 74 has the function of detecting the concentration of specific gas in air, the sensor 74 can be configured as e.g. an oxygen sensor. In the case where the sensor 74 has the function of detecting the concentration of specific gas in the air, the discharge control operation 4 may be configured to control the creeping discharge section 40 (discharge section) to increase the maximum voltage between the maximum voltage between the discharge electrode 42 (first electrode) and the ground electrode 44 (second electrode) with increase in the concentration of the specific gas detected by the sensor 74 (concentration detection section).

The higher the concentration of the air, the lower the concertation of the discharge gas. By the above control, the maximum voltage between the maximum voltage applied between the discharge electrode 42 (first electrode) and the ground electrode 44 (second electrode) is increased with decrease in the concentration of the discharge gas. Although the plasma discharge becomes difficult to perform due to decrease in the concentration of the discharge gas, such difficulty is compensated for by increasing the applied voltage in accordance with the degree of decrease of the discharge gas concentration.

In one embodiment example, the amplitude of the high-frequency voltage (i.e. maximum voltage) applied between the discharge electrode 42 (first electrode) and the ground electrode 44 (second electrode) is set to a first voltage V1 when the oxygen concentration detected by the sensor 74 is in a first range. When the oxygen concentration detected by the sensor 74 is in a second range higher than the first range, the amplitude of the high-frequency voltage (i.e. maximum voltage) applied between the discharge electrode 42 (first electrode) and the ground electrode 44 (second electrode) is set to a second voltage V2 higher than the first voltage V1. When the oxygen concentration detected by the sensor 74 is in a third range higher than the second range, the amplitude of the high-frequency voltage (i.e. maximum voltage) applied between the discharge electrode 42 (first electrode) and the ground electrode 44 (second electrode) is set to a third voltage V3 higher than the second voltage V3. By changing the maximum voltage stepwisely as mentioned above, the maximum voltage is controlled according to the concentration of the discharge gas. Although the maximum voltage is changed in three stages in this embodiment example, the maximum voltage may be changed in two stages or in four or more stages.

In another embodiment example, it is feasible to change the amplitude of the high-frequency voltage (i.e. maximum voltage) between the discharge electrode 42 (first electrode) and the ground electrode 44 (second electrode) according to a linear expression such as Y = A × X + b where X is the oxygen concentration detected by the sensor 74; and Y is the amplitude of the high-frequency voltage (i.e. maximum voltage), or any other arithmetic expression, such that the maximum voltage is increased with increase in the concentration of the specific gas.

This function may be imparted to the plasma irradiation apparatus 20 of the first embodiment.

Although the sensor 74 is configured as an oxygen sensor in the above embodiment example, the sensor 74 may alternatively be configured as a nitrogen sensor.

### <Other Embodiments>

The present invention is not limited to the configurations of the embodiments described above with reference to the drawings. For example, the features of some of the embodiments may be combined so long as they are not contradictory to one another. Furthermore, the following examples also fall within the technical scope of the present invention.

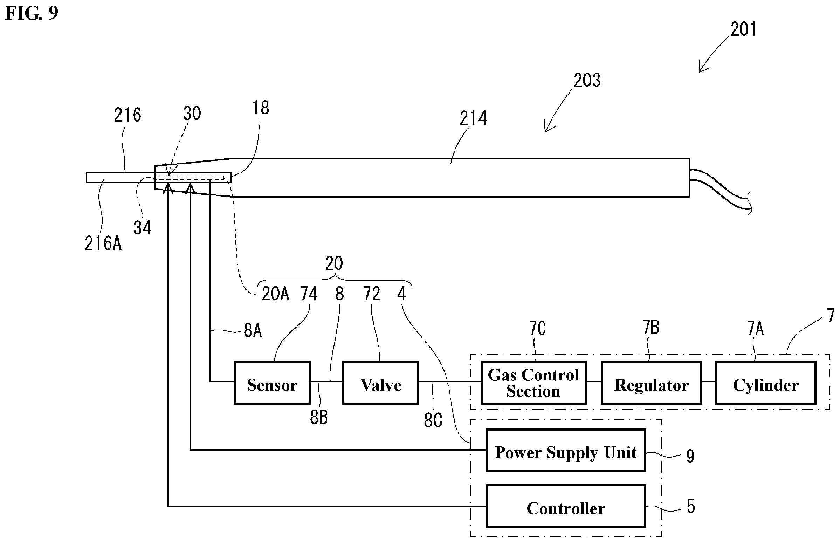

Although the distal device 3 functions as an ultrasonic scalpel in each of the first and second embodiments, there may be embodied a surgical system 201 as shown in FIG. 9 in which a distal device 203 with an electric scalpel function installs therein a plasma irradiation apparatus 20 as in the case of the first and second embodiments. The plasma irradiation apparatus 20 installed in the distal device 203 is similar in function and configuration to those of the first and second embodiments. The distal device 203 includes: an acting member 216 having an acting portion 216Athat acts on biological tissue; and a controller 5 having not only the same functions as those of the first and second embodiments but also the function of supplying a high-frequency current to the acting member 216. The acting member 216 is made of e.g. a metal material in a shaft shape, and functions an electrode part through which the high-frequency current supplied from the controller 5 flows. The acting member 216 performs the function of a known electric scalpel to perform incision action, ablation action or thermocoagulation hemostasis action on the target substance (biological tissue) by the flow of the high-frequency current through the acting member 216 (electrode part). It is thus possible, through the use of the common distal device 203, to perform incision treatment, ablation treatment or thermocoagulation hemostasis treatment on the biological tissue by flow of high-frequency current through the acting member 216 and to perform minimally invasive hemostasis treatment on the biological tissue by irradiation with low-temperature plasma from the plasma irradiation apparatus 20.

In the first and second embodiments, the distal device is provided with an ultrasonic knife function by installing therein the plasma irradiation apparatus. There may alternatively be provided a distal device by assembling the plasma irradiation apparatus to any known surgical instrument (such as scalpel, forceps etc.) with no electrical function.

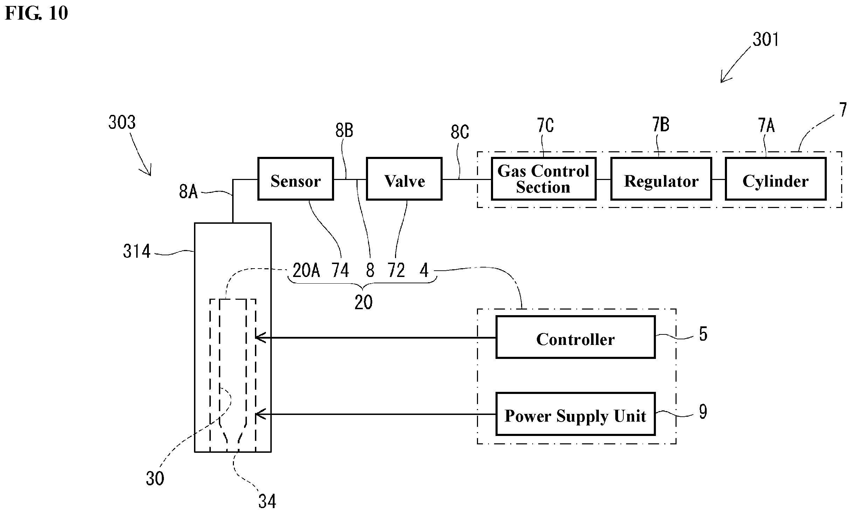

Although the plasma irradiation apparatus is installed as a part of the distal device in the surgical system in each of the first and second embodiments, the plasma irradiation apparatus is not necessarily installed in the distal device. For example, there may be embodied a plasma irradiation system 301 as shown in FIG. 10. This plasma irradiation system 301 is applicable as a surgical system or applicable for any use other than surgical use. In the embodiment example of FIG. 10, a distal device 303 is provided in which a plasma generator 20A similar to that of the first embodiment is installed in a casing 314, with the casing 314 and the plasma generator 20A being integrated as a grip unit (hand grip part).

Although the controller 5 is configured to control not only the drive section (such as ultrasonic vibration section 12) but also the power supply unit 9 in the above-mentioned embodiments, a controller for controlling the drive section (such as ultrasonic vibration section 12) and a controller for controlling the power supply unit 9 may be provided separately.

In the above embodiments, the creeping discharge section 40 is exemplified as one example of the discharge section. In an alternative embodiment, the discharge section may be of any other discharge type capable of performing plasma discharge. For example, the discharge section may alternatively be provided as a space discharge section in which a space and a dielectric layer are interposed between two electrodes so as to perform plasma discharge (space discharge).

Although the valve 72 is configured as a check valve and arranged in the gas supply channel 8 in the first embodiment, the valve configured as a check valve may alternatively be arranged in the gas guide channel 30.

Although the valve 72 is configured as mentioned above and arranged in the gas supply channel 8 in the second embodiment, the valve configured as mentioned above may alternatively be arranged in the gas guide channel 30.

As one example of the arrangement configuration of the valve in the gas flow pathway, the valve 72 is arranged in the gas supply channel 8 (that is, the portion of the gas flow pathway downstream of the gas control section 7C) in the second embodiment. However, the arrangement configuration of the valve is not limited to this example. For example, a valve similar to the valve 72 of the second embodiment may be arranged in a portion of the gas flow pathway upstream of the gas control section 7C).

In the second embodiment, the valve 72 (valve) is intermittently opened at a smaller opening degree during the rest period from the end of the discharge period to the start of the next preparation period (in FIG. 8, the period from time td to time tf) than that during the discharge period. The valve opening control is however not limited to such an example. For example, the valve 72 (valve) may be continuously opened at a smaller opening degree during the rest period from the end of the discharge period to the start of the next preparation period (in FIG. 8, the period from time td to time tf) than that during the discharge period. It means that the stop periods T31 and T32 may not provided in the example of FIG. 8.

Although the valve 72 is configured as a check valve in the first embodiment and configured as a valve in the second embodiment, these configurations may be used in combination. For example, a check valve with the same function as the valve 72 of the first embodiment may be arranged on an upstream or downstream side of the valve 72 (valve) in the configuration of the second embodiment.

In the above-described embodiments, the drive section is disposed inside of the casing which constitutes a part of the distal device (more specifically, the casing in which the acting member is installed). Alternatively, the drive section may be disposed outside of the casing. Even in the case where the drive section is disposed outside of the casing, the drive section can be regarded as a part of the distal device.

In the claims and specification, the expression "acting on biological tissue" means that the acting member exerts an influence on the biological tissue to perform at least one of incision treatment, ablation treatment and hemostasis treatment. The acting members exemplified in the above-described embodiments are merely examples. The acting member can employ various structures other than those in the above-described embodiments as long as the acting member exerts an influence on the biological tissue to perform at least one of incision treatment, ablation treatment and hemostasis treatment.

### Description of Reference Numerals

- 3, 203, 903:: Distal device
- 4:: Discharge control section
- 5:: Controller (Valve control section)
- 7C:: Gas control section
- 8:: Gas supply channel
- 20:: Plasma irradiation apparatus
- 30:: Gas guide channel
- 32:: Inlet port
- 34:: Outlet port
- 36:: Flow path
- 40:: Creeping discharge section (Discharge section)
- 42:: Discharge electrode (First electrode)
- 44:: Ground electrode (Second electrode)
- 51:: First dielectric layer (Dielectric layer)
- 72:: Valve (Check valve or Valve)
- 74:: Sensor (Concentration detection section)

## Claims

1. A plasma irradiation apparatus comprising:
a gas guide channel defining therein a flow path for flow of a discharge gas, with an outlet port formed at an end of the flow path;
a discharge section having a first electrode, a second electrode and a dielectric layer interposed between the first electrode and the second electrode and being operable to generate plasma discharge in the gas guide channel; and
a gas supply channel defining therein a path for supply of the discharge gas to the gas guide channel, the gas supply channel being disposed at a location closer to the gas guide channel than a gas control section which executes flow rate control of the discharge gas,
the plasma irradiation apparatus being adapted for mounting on a distal device which is movable relative to a target substance,
wherein the plasma irradiation apparatus further comprises a check valve arranged in the gas guide channel or the gas supply channel to allow the discharge gas to flow in a first direction toward the outlet port and prevent the discharge gas from flowing in a second direction reverse to the first direction.

2. A plasma irradiation apparatus, comprising:
a gas guide channel defining therein a flow path for flow of a discharge gas, with an outlet port formed at an end of the flow path;
a discharge section having a first electrode, a second electrode and a dielectric layer interposed between the first electrode and the second electrode and being operable to generate plasma discharge in the gas guide channel; and
a gas supply channel defining therein a path for supply of the discharge gas to the gas guide channel,
the plasma irradiation apparatus being adapted for mounting on a distal device which is movable relative to a target substance,
wherein the plasma irradiation apparatus further comprises:
a valve arranged in the gas guide channel or the gas supply channel to control a flow rate of the discharge gas according to an opening degree of the valve;
a discharge control section that controls the discharge section to perform the plasma discharge; and
a valve control section that controls opening and closing of the valve, and
wherein the valve control section is configured to:
keep the valve opened during at least a discharge period in which the discharge control section allows the discharge section to perform the plasma discharge; and
open the valve during a predetermined preparation period, in which the discharge control section prohibits the discharge section from performing the plasma discharge, before the discharge period.

3. The plasma irradiation apparatus according to claim 2,
wherein the valve control section is configured to open the valve at a larger opening degree during at least a partial period in the preparation period than that during the discharge period.

4. The plasma irradiation apparatus according to claim 2 or 3,
wherein the valve control section is configured to continuously or intermittently open the valve at a smaller opening degree during a rest period from an end of the discharge period to a start of a next preparation period than that during the discharge period.

5. The plasma irradiation apparatus according to any one of claims 2 to 4, further comprising a concentration detection section that detects a concentration of the discharge gas or specific gas in air in a pathway portion of the gas guide channel or the gas supply channel located closer to the outlet port than the valve.

6. The plasma irradiation apparatus according to claim 5,
wherein the concentration detection section detects the concentration of the discharge gas in the pathway portion, and
wherein the discharge control section is configured to:
allow the discharge section to perform the plasma discharge when the concentration of the discharge gas detected by the concentration detection section is higher than or equal to a threshold value; and
when the concentration of the discharge gas detected by the concentration detection section is lower than the threshold value, prohibit the discharge section from performing the plasma discharge.

7. The plasma irradiation apparatus according to claim 5,
wherein the concentration detection section detects the concentration of the specific gas; and
wherein the discharge control section is configured to increase a maximum voltage between the first and second electrodes with increase in the concentration of the specific gas detected by the concentration detection section.

8. A plasma irradiation method using a plasma irradiation apparatus, the plasma irradiation apparatus comprising: a gas guide channel defining therein a flow path for flow of a discharge gas, with an outlet port formed at an end of the flow path; a discharge section having a first electrode, a second electrode and a dielectric layer interposed between the first electrode and the second electrode and being operable to generate plasma discharge in the gas guide channel; and a gas supply channel defining therein a path for supply of the discharge gas to the gas guide channel, the plasma irradiation method comprising:
mounting the plasma irradiation apparatus to a distal device which is movable relative to a target substance;
arranging a valve in the gas guide channel or the gas supply channel;
operating a discharge control section to control the plasma discharge of the discharge section; and
operating a valve control section to: keep the valve opened during at least a discharge period in which the discharge control section allows the discharge section to perform the plasma discharge; and open the valve during a predetermined preparation period, in which the discharge control section prohibits the discharge section from performing the plasma discharge, before the discharge period.

9. The plasma irradiation apparatus according to claim 8,
wherein the valve control section is operated to open the valve at a larger opening degree during at least a partial period in the preparation period than that during the discharge period.

10. The plasma irradiation apparatus according to claim 8 or 9,
wherein the valve control section is operated to continuously or intermittently open the valve at a smaller opening degree during a rest period from an end of the discharge period to a start of a next preparation period than that during the discharge period.

11. The plasma irradiation apparatus according to any one of claims 8 to 10,
wherein the plasma irradiation apparatus comprises a concentration detection section that detects a concentration of the discharge gas in a pathway portion of the gas guide channel or the gas supply channel located closer to the outlet port than the valve, and
wherein the discharge control section is operated to: allow the discharge section to perform the plasma discharge when the concentration of the discharge gas detected by the concentration detection section is higher than or equal to a threshold value; and prohibit the discharge section from performing the plasma discharge when the concentration of the discharge gas detected by the concentration detection section is lower than the threshold value.

12. The plasma irradiation apparatus according to any one of claims 8 to 11,
wherein the plasma irradiation apparatus comprises a concentration detection section that detects a concentration of specific gas in air in a pathway portion of the gas guide channel or the gas supply channel located closer to the outlet port than the valve, and
wherein the discharge control section is operated to increase a maximum voltage between the first and second electrodes with increase in the concentration of the specific gas detected by the concentration detection section.
